# EUROPEAN PATENT SPECIFICATION

(11) **EP 3 536 774 B1**
(45) Date of publication and mention of the grant of the patent: **16.06.2021**
(21) Application number: 16920517.6
(22) Date of filing: 01.11.2016
(51) Int. Cl.: C12M 1/00, B01L 3/00, B01L 9/00

(54) **GENE SEQUENCING CHIP AND COMBINATION WITH MOUNTING FRAME**
GENSEQUENZIERUNGSCHIP UND KOMBINATION MIT MONTAGERAHMEN
PUCE DE SÉQUENÇAGE DE GÈNE ET COMBINAISON AVEC CADRE DE MONTAGE

(43) Date of publication of application: 11.09.2019
(73) Proprietor: MGI Tech Co., Ltd., Yantian District Shenzhen Guangdong 518083 (CN)
(72) Inventor: XU, Hong, Shenzhen Guangdong 518083 (CN); ZHANG, Songzhen, Shenzhen Guangdong 518083 (CN); DAI, Chongchong, Shenzhen Guangdong 518083 (CN); ZHENG, Huan, Shenzhen Guangdong 518083 (CN); GAN, Yong, Shenzhen Guangdong 518083 (CN); WANG, Jing, Shenzhen Guangdong 518083 (CN); NI, Ming, Shenzhen Guangdong 518083 (CN); TAN, Daxi, Shenzhen Guangdong 518083 (CN); ZHANG, Wenyu, Shenzhen Guangdong 518083 (CN)
(74) Representative: Hoffmann Eitle
(86) International application number: PCT/CN2016/104215
(87) International publication number: WO 2018/081920

(56) References cited:
- CN-A- 105 624 020
- CN-A- 105 772 125
- CN-U- 205 368 331
- US-A1- 2010 323 350
- US-A1- 2014 024 126

## Description

### FIELD

The disclosure relates to gene sequencing consumables, in particular to a gene sequencing chip and a mounting frame for the same.

### BACKGROUND

DNA sequencing, synonym for DNA ordering or gene sequencing, refers to analysis of the base order of a particular DNA fragment, i.e., the arrangement of adenine (A), thymine (T), cytosine (C) and guanine (G).

Currently, the Next-generation sequencing is a popular gene sequencing method on the market, conducted by loading a gene molecule to be sequenced onto a gene sequencing chip, followed by analysis of the gene sequence of the gene molecule to be sequenced via its fluorescent signal measured on a gene sequencing platform. A CN invention patent with the application No. 200680029826.4 and the publication No. CN101466847B has disclosed a method for loading a gene molecule to be sequenced onto a gene sequencing chip.

A CN application (with the application No. 201410625939.7 and the publication No. CN105624020A) discloses a microfluidic chip, actually a gene sequencing chip. A substrate 101 of the chip includes several microchannels 1011 which are divided by metal microsphere sealing strips 1013 and into which a liquid reagent is injected via a linker seat 103. The metal microsphere sealing strip 1013 dispensed on the substrate 101 is extruded to be spread out upon adhesion of the substrate 101 with a cover sheet 102, forming irregular edges and thus resulting in an irregular inner wall of the microchannel 1011, thereby interfering uniform flow of liquid. Further, the liquid reagent directly contacts the metal microsphere sealing strip 1013 to a large extent, which would incur a chemical reaction, thus leading an inaccurate sequencing result. Furthermore, the substrate 101 is not fixed by a sealant or seal ring which is deposited between an upper fixing plate 1055 and a lower fixing plate 1051 (which constitute the chip along with other elements), thus may cause poor sealing performance to the microchannel 1011.

The subject of gene sequencing, usually in molecular scale, is sensitive to flow rate, pressure and the like of a reagent. Therefore, the flow rate and stability of pressure of a reagent in a gene sequencing chip are important criteria for assessing quality of the gene sequencing chip. Based on the above, the microfluidic chip in CN105624020A still has many disadvantages, and a gene sequencing chip (i.e. a microfluidic chip) prepared by the method in CN101466847B cannot achieve an excellent result.

US20100323350A1 provides methods and compositions, including, without limitation, algorithms, computer readable media, computer programs, apparatus, and systems for determining the identity of nucleic acids in nucleotide sequences using, for example, data obtained from sequencing by synthesis methods. A plurality of smaller flow cells is employed, each with a relatively small area to be imaged, in order to provide greater flexibility and efficiency.

US2014/024126A1 disclosed a flow-channel device for detecting light emission, having a flow channel which is structured by bonding of at least two substrates, wherein a first substrate has a first groove which constitutes the flow channel, and a second groove which contains an adhesive therein that contains an organic material, and the second groove has a light-shielding film provided on an inner wall thereof.

Note: the drawings and the reference numerals of technical features mentioned in the background section refer to the drawings and reference numerals in the cited documents only, and do not refer to the drawings and reference numerals in the present application.

### SUMMARY

Embodiments of the present disclosure aim at solving at least one of the problems existing in the related art, such as non-uniform flow rate of a liquid in a gene sequencing chip, an inaccurate result by direct contact of the liquid with a sealant, poor sealing performance and the like. For this, the present disclosure in embodiments provides a gene sequencing chip with excellent performance, and a mounting frame used in cooperation with the gene sequencing chip.

The gene sequencing chip according to the invention includes the features of claim 1.

In some embodiments of the present disclosure, the sealant channel is filled with a sealant and is separated from the flow channel, avoiding contact of a liquid in the flow channel with the sealant to a maximum extent, thus preventing an inaccurate sequencing result incurred by the chemical reaction between the liquid and the sealant. Further, the flow channel is formed with a highly smooth inner surface to ensure uniform flow rate and stable pressure for the liquid, therefore improving accuracy of the sequencing results effectively. Furthermore, the flow channel is enclosed only by the flow channel groove and the silicon wafer such that the flow channel has few contacting surfaces, thereby providing excellent sealing performance.

In some embodiments of the present disclosure, the first and second terminal sections each are in a shape of sharp angle protruding outward.

In accordance with the present invention, the silicon wafer is further provided with a boss which is located at the middle section and on which the adaptor region is provided.

In one embodiment of the present disclosure, the gene sequencing chip is optimized as follows. The sharp angle protruding outward is an included angle of 60° and chamfered with a corner radius of 2 mm; the first and second terminal sections each are connected to the middle section through a rounded transition with a corner radius of 2 mm; the flow channel is of a length of 69.8 mm, a width of 5 mm for the middle section, and a distance from an upper surface of the boss to a bottom surface of the flow channel groove of 0.05±0.005 mm; and the liquid inlet and the liquid outlet, each formed as a round hole in a diameter of 0.8 mm, are apart from each other at a center distance of 69 mm.

To further improve the resistance to external force for the gene sequencing chip, in one embodiment of the present disclosure, the transparent cover sheet is further formed with a reinforcing agent groove at the surface where the transparent cover sheet is adhered to the silicon wafer, the reinforcing agent groove and the silicon wafer enclose a reinforcing agent channel which is filled with a reinforcing agent and surrounds the sealant channel, and the silicon wafer is further provided with an air hole which is located between the reinforcing agent channel and the sealant channel. In some embodiments of the present disclosure, the sealant and the reinforcing agent are selected from polyurethane adhesive, hot melt adhesive and ultraviolet curing adhesive.

In one embodiment of the present disclosure, the adaptor region is in a round shape with a diameter of 220 nm, and the silicon wafer is provided with a plurality of the adaptor regions arranged in an array with a center distance of 900 nm between adjacent two adaptor regions.

In another aspect of the present disclosure, provided in embodiments is a mounting frame for the gene sequencing chip as described above, including:
a mounting groove, configured to accommodate and fit to the gene sequencing chip and provided with an insertion opening for receiving the gene sequencing chip at one end of the mounting groove;
an anti-off structure, arranged at the insertion opening and configured to prevent the gene sequencing chip from slipping out of the insertion opening;
an open region, located right above the mounting groove and configured to allow observation of the flow channel of the gene sequencing chip accommodated in the mounting groove from outside; and
a positioning hole, configured to allow the mounting frame to be positioned on an exterior platform.

The mounting frame for the gene sequencing chip provided in embodiments of the present disclosure can be useful in protecting the gene sequencing chip, as well as for assisting the positioning of the gene sequencing chip on an exterior platform.

To further improve stability of the gene sequencing chip mounted, the mounting groove is provided with symmetric protrusions at inner side walls which are configured to restrict the gene sequencing chip within the mounting groove.

In a particular embodiment, the anti-off structure includes first and second elastic pieces which are symmetrically arranged at the insertion opening, wherein the first elastic piece has first and second ends, the second elastic piece has third and fourth ends, the first and third ends are fixed at two sides of the insertion opening, respectively, at a distance greater than the width of the gene sequencing chip; the second and fourth ends are movable with a reset distance smaller than the width of the gene sequencing chip, the first and third ends are located at an upstream end along an insertion direction, and the second and fourth ends are located at a downstream end along the insertion direction.

### BRIEF DESCRIPTION OF THE DRAWINGS

Figure 1 is a schematic stereogram of a gene sequencing chip in one embodiment of the present disclosure;
Figure 2 is a schematic diagram of Figure 1 where a transparent cover sheet is not displayed;
Figure 3 is a decompose diagram of a gene sequencing chip in one embodiment of the present disclosure;
Figure 4 is a schematic diagram of a bottom view of Figure 3;
Figure 5 is an enlarged partial view for part I in Figure 4;
Figure 6 is a schematic stereogram of a mounting frame in one embodiment of the present disclosure, which is equipped with a gene sequencing chip of which a transparent cover sheet is not displayed;
Figure 7 is a decompose diagram of Figure 6 where the transparent cover sheet of the gene sequencing chip is displayed;
Figure 8 is a schematic diagram of a bottom view of Figure 6;
Figure 9 is a schematic diagram of a bottom view of Figure 7;
Figure 10 is a sectional view taken along the line A-A in Figure 6 where a transparent cover sheet is displayed;
Figure 11 is an enlarged partial view for part II in Figure 10;
Figure 12 is a sectional view taken along the line B-B in Figure 6 where a transparent cover sheet is displayed;
Figure 13 is an enlarged partial view for part III in Figure 12;
Figure 14 is a schematic diagram of a mounting frame in another embodiment of the present disclosure, which is equipped with a gene sequencing chip;
Figure 15 is a schematic diagram of the gene sequencing chip of Figure 14 where a transparent cover sheet is not displayed;
Figure 16 is an enlarged view for an adaptor region in one embodiment of the present disclosure;
Figure 17 is a schematic diagram showing a velocity field of fluid in a gene sequencing chip in one embodiment of the present disclosure;
Figure 18 is a schematic diagram showing pressure of fluid in a gene sequencing chip in one embodiment of the present disclosure.

### DETAILED DESCRIPTION

To explicitly illustrate the objects, technical solutions and advantages of the present disclosure, the present disclosure is further described in detail below in combination with the drawings and examples. It is understood that the specific examples described herein are explanatory, and cannot be construed to limit the scope of the present disclosure.

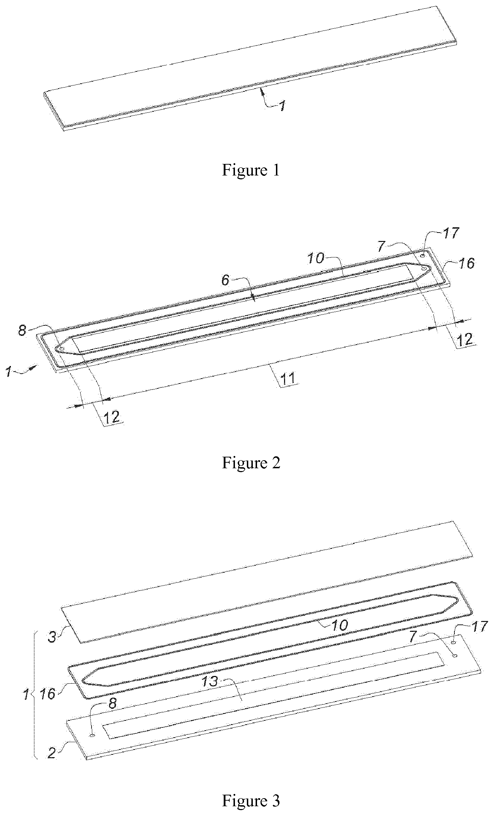

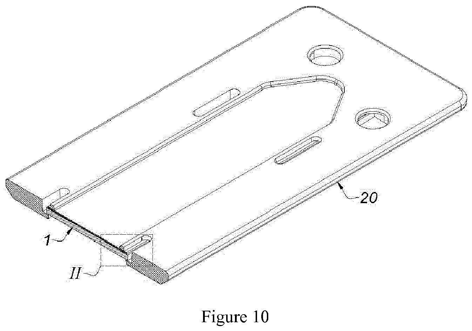

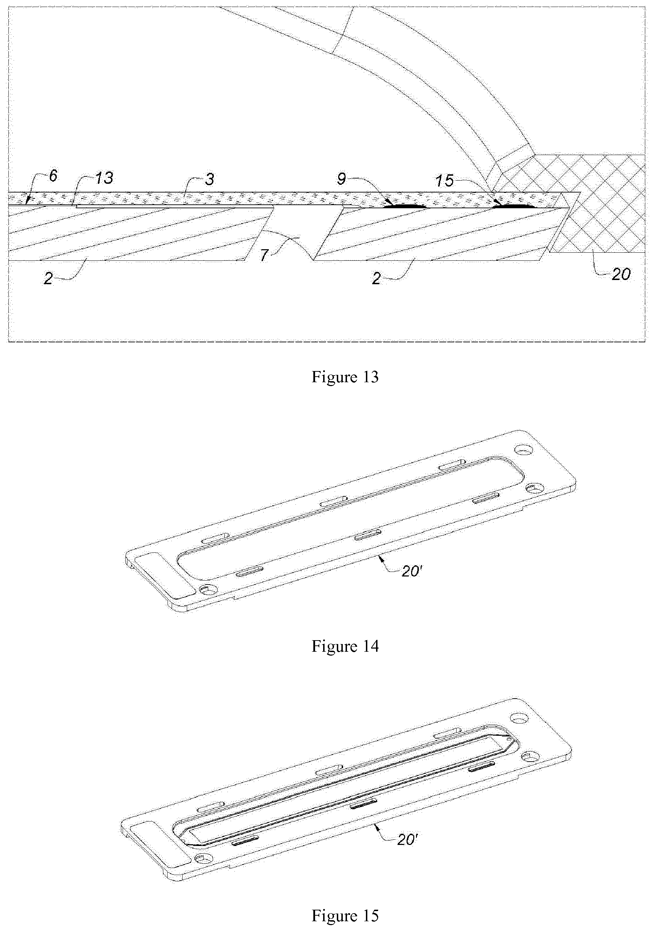

The gene sequencing chip and the mounting frame for the same provided in embodiments of the present disclosure can have various embodiments. For a better understanding, the following embodiments are described by taking one of the embodiments as a main line, and Figures 1 to 13 are the schematic diagrams of the gene sequencing chip and the mounting frame for the same described in accordance with the main line. Alternatives of technical features of the embodiment as the main line are interspersed as branches when the embodiment is described, and the alternatives which can be clearly expressed literally will not be illustrated by drawings. Figures 14 and 15 are schematic diagrams of another embodiment of a mounting frame for a gene sequencing chip.

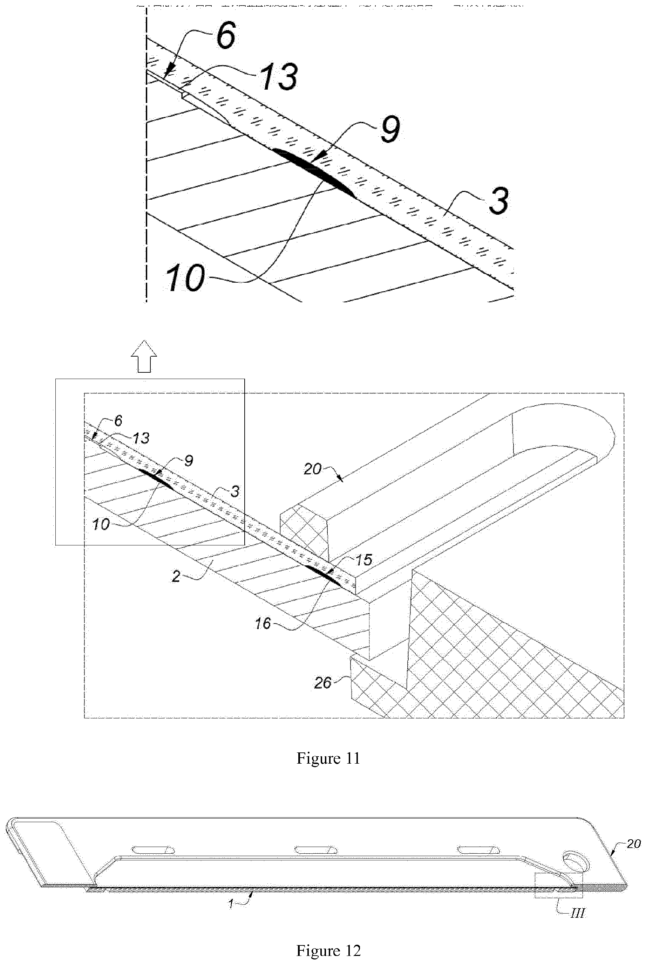

In one embodiment of the present disclosure, referring to Figures 1 to 5, the gene sequencing chip 1 includes a silicon wafer 2 and a transparent cover sheet 3. The silicon wafer 2 is provided with an adaptor region containing an adaptor which is capable of capturing a gene molecule to be sequenced, and a liquid inlet 7 and a liquid outlet 8 connecting a flow channel 6 with outside. The transparent cover sheet 3 is adhered to the silicon wafer 2 and formed with a flow channel groove 4 and a sealant groove 5 at a surface where the transparent cover sheet 3 is adhered to the silicon wafer 2. The flow channel groove 4 and the silicon wafer 2 enclose the flow channel 6 which allows a liquid to flow through and accommodates the adaptor region, referring to Figures 11 and 13 for understanding. The sealant groove 5 and the silicon wafer 2 enclose a sealant channel 9 which is filled with a sealant 10 and surrounds the flow channel 6, also referring to Figures 11 and 13 for understanding.

The transparent cover sheet 3 is made from a light-transmitting material, with excellent light transmittance, allowing an exterior laser to penetrate through it and radiate the flow channel 6, as well as allowing the fluorescent signal in the flow channel 6 to be emitted outside, which can be photographed clearly by an exterior camera.

The adaptor region is chemically modified by seeding a specific gene fragment as an adaptor onto its surface, thus will capture a gene molecule to be sequenced when a solution containing the gene molecule to be sequenced flows through, with the gene molecule fixed onto the adaptor region. The adaptor region of the present disclosure can be modified by various methods, and accordingly the gene molecule to be sequenced can be captured by various methods, for example, in one embodiment of the present disclosure, the modification of the adaptor region and capturing of the gene molecule to be sequenced are conducted according to the methods disclosed in CN invention patent CN101466847B, from which such the gene molecule to be sequenced is called as "DNA nanoball" (DNB for short).

In one embodiment of the present disclosure, the sealant channel 9 is filled with a sealant 10 and is separated from the flow channel 6, avoiding contact of a liquid in the flow channel 6 with the sealant 10 to a maximum extent, thus preventing an inaccurate sequencing result incurred by the chemical reaction between the liquid and the sealant 10. Further, the flow channel 6 is formed with a highly smooth inner surface to ensure uniform flow rate and stable pressure for the liquid, therefore improving accuracy of the sequencing results effectively. Furthermore, the flow channel 6 is enclosed only by the flow channel groove 4 and the silicon wafer 2 such that the flow channel 6 has few contacting surfaces, thereby providing excellent sealing performance.

The gene sequencing chip 1 prepared can achieve high sequencing throughput although in a small size. The flow channel 6 can be formed with an extremely smooth inner surface by the current technology, benefiting uniform flow of liquid and avoiding generation of bubbles.

Referring to Figure 2 for understanding, in one embodiment of the present disclosure, the flow channel 6, in a long strip shape, includes a middle section 11, first and second terminal sections 12. The first and second terminal sections 12 are located at and connected to two ends of the middle section 11, respectively. The first and second terminal sections 12 each are in a shape of sharp angle protruding outward. The liquid inlet 7 and the liquid outlet 8 are located at the first and second terminal sections 12, respectively.

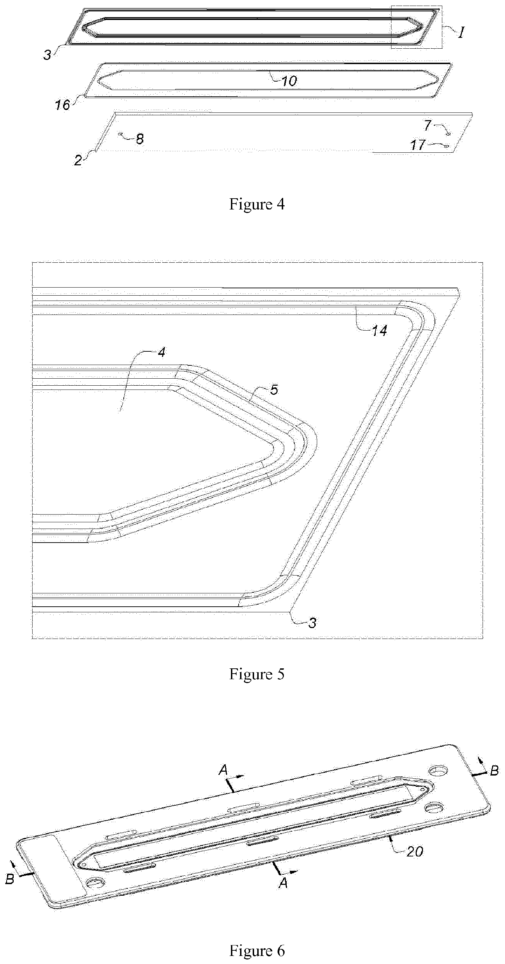

Figure 2 is a schematic diagram of the genetic sequencing chip 1 of which the transparent cover sheet 3 is not displayed, and the shape of the sealant 10 therein substantially represents the shape of the flow channel 6, which is good for the skilled in the art to understand the embodiment of the present disclosure. It should be noted, the flow channel 6 in the embodiment is surrounded by the sealant 10 at a certain interval with the transparent cover sheet 3 as an separator therebetween (referring to Figures 5, 11 and 13), where the certain interval is extremely close, such that the shape of the flow channel 6 can be substantively represented by the shape of the sealant 10. The flow channel 6 in such configuration further ensures uniform flow rate and stable pressure of a liquid. With fluid mechanics analysis and lots of trial results, it is found that the fluid velocity is most uniform and the pressure is best in the middle section 11 throughout the entire flow channel 6, thus the adaptor region is usually arranged at the middle section 11.

In another embodiment of the present disclosure, for example, the silicon wafer 2 is further provided with a boss 13 which is located at the middle section 11 and on which the adaptor region is provided. Referring to Figures 11 and 13, the distance between the adaptor region and the bottom surface of the flow channel groove 4 is shortened by provision of the boss 13, thus reducing the internal space of the flow channel 6, the reagent consumption and sequencing cost; as well shortening the optical path of light wave in the flow channel 6 and the optical path difference of fluorescence in different wavelengths passing through the transparent cover sheet 3, therefore accuracy of fluorescent signal collected with exterior detection devices (such as a camera) can be further improved.

Generally, prior to adhering of the transparent cover sheet 3 on the silicon wafer 2, a liquid sealant which would be solidified to be the sealant 10 is injected into the sealant groove 5. However, it is hardly to ensure that the liquid sealant is injected in an exact amount such that the sealant channel 9 is just completely filled up, with a slight amount of sealant spilling out and flowing into the space between the transparent cover sheet 3 and the silicon wafer 2, which is at a risk of contacting the liquid in the flow channel 6 (usually occurring in an extremely low chance, but unavoidable), even resulting in a chemical reaction, thereby affecting the accuracy of sequencing results. For this, a boss 13 is provided, referring to Figures 11 and 13, where the adaptor region is arranged on the boss 13 (that is located on the upper surface of the boss 13), which is higher than the contacting surface between the transparent cover sheet 3 and the silicon wafer 2 under the upper surface of the boss 13 (that is located lower than the upper surface of the boss 13), thereby generating a height difference between the adaptor region and the contacting surface, thus the adaptor region arranged at a higher position cannot be affected, even if the liquid contacts with the sealant at a lower position (since the possible resulting product will not flow up to the upper surface of the boss 13 under the gravity), such that the accuracy of the sequencing results will be guaranteed.

With fluid mechanics analysis and lots of trial results, it is found that the flow channel 6 can be optimized as follows. The sharp angle protruding outward of the first and second terminal sections 12 is an included angle of 60° and chamfered with a corner radius of 2 mm; the first and second terminal sections 12 each are connected to the middle section 11 through a rounded transition with a corner radius of 2 mm; the flow channel 6 is of a length of 69.8 mm, a width of 5 mm for the middle section 11, and a distance from an upper surface of the boss 13 to a bottom surface of the flow channel groove 4 of 0.05±0.005 mm; and the liquid inlet 7 and the liquid outlet 8, each formed as a round hole in a diameter of 0.8 mm, are apart from each other at a center distance of 69 mm.

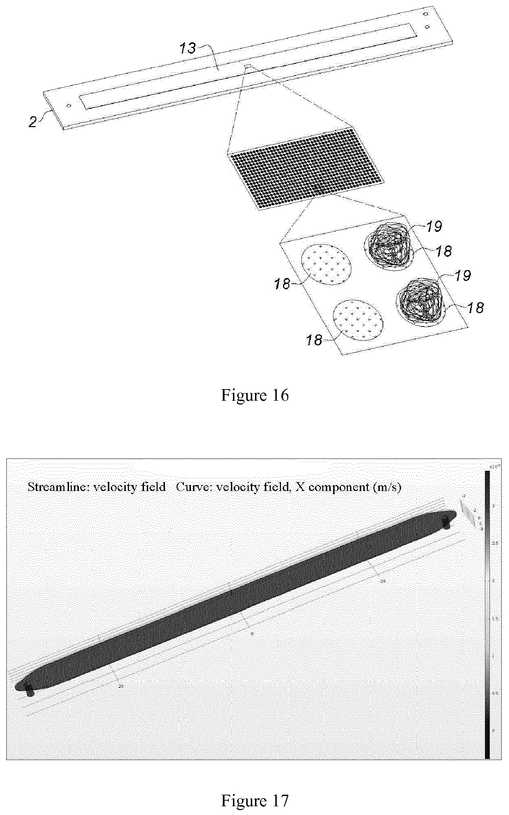

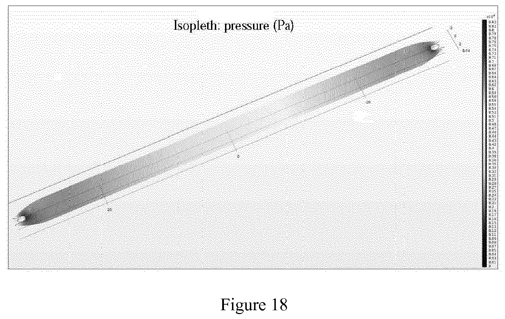

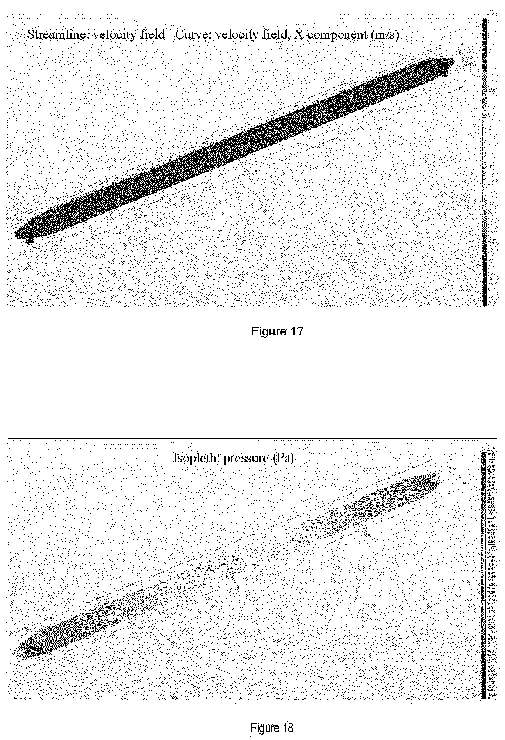

Figures 17 and 18 are diagrams showing the test results of flow channel optimized as above. Figure 17 in which the liquid inlet is at the right end and the liquid outlet is at the left end, shows a highly uniform flow field of the flow channel 6, with very uniform flow velocity at each position. Figure 18 in which the liquid inlet is at the right end and the liquid outlet is at the left end, shows a highest pressure near the liquid inlet and a lowest pressure near the liquid outlet, with a uniform pressure decreasing during the middle section. In this trial, the liquid flow is driven under a negative pressure provided at the liquid outlet.

In one embodiment of the present disclosure, referring to Figures 5, 11 and 13, to further improve the resistance to external force for the gene sequencing chip 1 and enhance the adhesive strength between the silicon wafer 2 and the transparent cover sheet 3, the transparent cover sheet 3 is further formed with a reinforcing agent groove 14 at the surface where the transparent cover sheet 3 is adhered to the silicon wafer 2, the reinforcing agent groove 14 and the silicon wafer 2 enclose a reinforcing agent channel 15 which is filled with a reinforcing agent 16 and surrounds the sealant channel 9, and the silicon wafer 2 is further provided with an air hole 17 which is located between the reinforcing agent channel 15 and the sealant channel 9. The air hole 17 can be useful in discharging the air between the reinforcing agent channel 15 and the sealant channel 9, as well useful in positioning during encapsulation of the gene sequencing chip.

The sealant and the reinforcing agent in embodiments of the present disclosure can be selected from polyurethane adhesive, hot melt adhesive, ultraviolet curing adhesive and other suitable adhesives. The sealants as described above can endure high temperature, thus the gene sequencing chip where the sealant is used for adhesion is capable of resisting deformation strongly under the high temperature, and exhibiting excellent sealing performance.

In one embodiment of the present disclosure, referring to Figure 16, the modification of the adaptor region at the silicon wafer and the capturing of the gene molecule to be sequenced is conducted according to the methods disclosed in CN invention patent CN101466847B. The adaptor region 18 is in a round shape with a diameter of 220 nm, and the silicon wafer 2 is provided with a plurality of the adaptor regions 18 (about 200 million) arranged in an array with a center distance of 900 nm between adjacent two adaptor regions. The adaptor region 18 is seeded with an adaptor, which can capture the gene molecule to be sequenced 19 (i.e. DNB in a diameter of about 200 nm), where one DNB can be specifically bond to one adaptor region 18. When the adaptor region is arranged as the size and adjacent distance as described above, clear fluorescent signals emitted can be collected within a resolution range acceptable for a camera, according to current technology.

In one embodiment of the present disclosure, referring to Figures 6 to 13, the mounting frame 20 provided can be configured to protect the gene sequencing chip 1, as well as to assist the positioning of the gene sequencing chip 1 on an exterior platform, where the mounting frame 20 includes:
a mounting groove 21, configured to accommodate and fit to the gene sequencing chip 1 and provided with an insertion opening 22 for receiving the gene sequencing chip 1 at one end of the mounting groove 21;
an anti-off structure 23, arranged at the insertion opening 22 and configured to prevent the gene sequencing chip 1 from slipping out of the insertion opening 22;
an open region 24, located right above the mounting groove 21 and configured to allow observation of the flow channel 6 of the gene sequencing chip 1 accommodated in the mounting groove 21 from outside; and
a positioning hole 25, configured to allow the mounting frame 20 to be positioned on an exterior platform,
in which the mounting frame 20 is formed as a hollow structure, and
the exterior platform is a customized gene sequencer.

In one embodiment of the present disclosure, the mounting groove 21 may be formed as a groove structure with a sealed bottom. In another embodiment, for simplification of structure, material-saving and facilitation of injection molding, the mounting groove 21 is formed as a groove structure with an open bottom, and provided with six symmetric protrusions 26 at two inner side walls (that is three protrusions at each inner side wall). The protrusions are configured to restrict the gene sequencing chip 1 within the mounting groove 21, and to restrain the deformation of the mounting frame 20. In still another embodiment of the present disclosure, the number and size of the protrusions 26 can be set as desired, for example, four protrusions 26 are set, with two protrusions at each inner side wall.

The mounting frame 20 is further provided with six fabrication holes 29, which are located right above the six protrusions 26 respectively and configured to allow the protrusions 26 to be injection molded more conveniently.

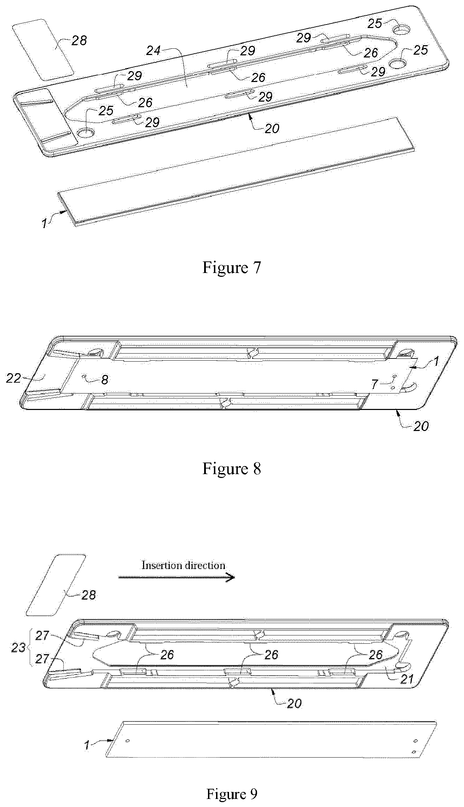

In a particular embodiment, referring to Figure 9, the anti-off structure 23 includes first and second elastic pieces 27 which are symmetrically arranged at the insertion opening 22, more particularly, the first elastic piece 27 has first and second ends, the second elastic piece 27 has third and fourth ends, the first and third ends are fixed at two sides of the insertion opening 22, respectively, at a distance greater than the width of the gene sequencing chip 1; the second and fourth ends are movable with a reset distance smaller than the width of the gene sequencing chip 1, the first and third ends are located at an upstream end along an insertion direction, and the second and fourth ends are located at a downstream end along the insertion direction. The anti-off structure in such configuration is good for loading and unloading the gene sequencing chip 1, and facilitates the gene sequencing chip 1 to be fixed in the mounting groove 21 firmly.

In one embodiment, referring to Figures 6 and 7, for example, the open region 24 is configured to conform to the shape of the flow channel 6 (with its area slightly bigger than that of the flow channel 6) and is located right above the flow channel 6, which allows the entire flow channel 6 to be observed from outside. In another embodiment, referring to Figures 14 and 15, the open region of a mounting frame 20' is formed as a long strip shape with round corners, which is slightly different from the open region 24, and is configured to allow the entire flow channel 6 to be completely fallen into the range of the open region of the mounting frame 20', thus the entire flow channel 6 can be observed from outside as well.

The mounting frame 20 can be further attached with a label 28, which is printed with a barcode or a two-dimensional code and the like, so as to facilitate the tracking and management of the gene sequencing chip and the mounting frame for the same, thus improving efficiency and accuracy of gene sequencing.

The gene sequencing chip and the mounting frame for the same provided in embodiments of the present disclosure are formed as individual simple structures with few components, thus can be easily assembled and produced.

To enable those skilled in the art to better understand the present disclosure, one implementation of the gene sequencing chip 1 and the mounting frame 20 for the same is exemplified briefly as the following steps, which is not intended to limit the protection scope of the present disclosure.

Step1. The silicon wafer 2 of the gene sequencing chip 1 is provided with more than 200 million of adaptor regions 18 seeded with adaptors via chemical modification on the surface of the silicon wafer 2, according to the methods disclosed in the CN invention patent CN101466847B.

Step 2. The gene sequencing chip 1 in step 1 is mounted into the mounting frame 20, which is subsequently placed on an exterior gene sequencer.

Step 3. A solution containing a gene molecule to be sequenced 19 (i.e. DNB) is injected into the flow channel 6 via the liquid inlet 7, with the DNB flowing through captured by and adhered to an adaptor on the adaptor region 18.

Step 4. A gene sequencing reagent which would react with the DNBs is injected into the cleaned flow channel 6, with a fluorescent signal generated which is subsequently collected by the gene sequencer, thus obtaining the sequences of the gene molecule to be sequenced.

During gene sequencing as described above, Combinatorial Probe-Anchor Synthesis (cPAS) is used, however it does not belong to the protection scope of this present disclosure and will not be described in detail herein. It should be noted, the chemical modification of the adaptor region on the silicon wafer and gene sequencing can be conducted with other methods in the present disclosure, which are not limited to the methods disclosed in CN101466847B as an example.

The description above is one embodiment of the present disclosure only, and is not intended to limit the present disclosure. The scope of the invention is defined by the appended claims.

## Claims

1. A gene sequencing chip (1), comprising a silicon wafer (2) and a transparent cover sheet (3) adhered to the silicon wafer (2), the silicon wafer (2) is provided with
an adaptor region (18) containing an adaptor which is capable of capturing a gene molecule to be sequenced, and
a liquid inlet (7) and a liquid outlet (8) connecting a flow channel (6) with outside;
said gene sequencing chip (1) is **characterized in that**:
the transparent cover sheet (3) is formed with a flow channel groove (4) and a sealant groove (5) at a surface where the transparent cover sheet (3) is adhered to the silicon wafer (2),
wherein the flow channel groove (4) and the silicon wafer (2) enclose the flow channel (6) which allows a liquid to flow through and accommodates the adaptor region (18), and
wherein the sealant groove (5) and the silicon wafer (2) enclose a sealant channel (9) which is filled with a sealant (10) and surrounds the flow channel (6),
wherein the flow channel (6), in a long strip shape, comprises a middle section (11) and first and second terminal sections (12) located at and connected to two ends of the middle section (11), respectively; wherein the liquid inlet (7) and the liquid outlet (8) are located at the first and second terminal sections (12), respectively; and wherein
the silicon wafer (2) is further provided with a boss (13) located at the middle section (11) of the flow channel (6) and on which the adaptor region (18) is provided.

2. The gene sequencing chip (1) according to claim 1, wherein
the first and second terminal sections (12) each are in a shape of sharp angle protruding outward.

3. The gene sequencing chip (1) according to claim 2, wherein
the sharp angle protruding outward is in an included degree of 60° and chamfered with a corner radius of 2 mm;
the first and second terminal sections (12) each are connected to the middle section (11) through a rounded transition with a corner radius of 2 mm;
the flow channel (6) is of a length of 69.8 mm, a width of 5 mm for the middle section (11), and a distance from an upper surface of the boss (13) to a bottom surface of the flow channel groove (4) of 0.05±0.005 mm; and
the liquid inlet (7) and the liquid outlet (8), each formed as a round hole in a diameter of 0.8 mm, are apart from each other at a center distance of 69 mm.

4. The gene sequencing chip (1) according to any one of claims 1 to 3, wherein
the transparent cover sheet (3) is further formed with a reinforcing agent groove (14) at the surface where the transparent cover sheet (3) is adhered to the silicon wafer (2),
the reinforcing agent groove (14) and the silicon wafer (2) enclose a reinforcing agent channel (15) which is filled with a reinforcing agent (16) and surrounds the sealant channel (9), and
the silicon wafer (2) is further provided with an air hole (17) which is located between the reinforcing agent channel (15) and the sealant channel (9).

5. The gene sequencing chip (1) according to claim 4, wherein the sealant (10) and the reinforcing agent (16) are selected from polyurethane adhesive, hot melt adhesive and ultraviolet curing adhesive.

6. The gene sequencing chip (1) according to any one of claims 1 to 5, wherein
the adaptor region (18) is in a round shape with a diameter of 220 nm, and
the silicon wafer (2) is provided with a plurality of the adaptor regions (18) arranged in an array with a center distance of 900 nm between adjacent two adaptor regions (18).

7. A combination of a mounting frame (20) for the gene sequencing chip (1) and the gene sequencing chip (1) according to any one of claims 1 to 6, being **characterized by**
a mounting groove (21), configured to accommodate and fit to the gene sequencing chip (1) and provided with an insertion opening (22) for receiving the gene sequencing chip (1) at one end of mounting groove (21);
an anti-off structure (23), arranged at the insertion opening (22) and configured to prevent the gene sequencing chip (1) from slipping out of the insertion opening (22);
an open region (24), located right above the mounting groove (21) and configured to allow observation of the flow channel (6) of the gene sequencing chip (1) accommodated in the mounting groove (21) from outside; and
a positioning hole (25), configured to allow the mounting frame (20) to be positioned on an exterior platform.

8. The combination according to claim 7, wherein the mounting groove (21) is provided with symmetric protrusions (26) at inner side walls which are configured to restrict the gene sequencing chip (1) within the mounting groove (21).

9. The combination according to claim 7 or 8, wherein
the anti-off structure (23) comprises first and second elastic pieces (27) which are symmetrically arranged at the insertion opening (22),
the first elastic piece (27) has first and second ends,
the second elastic piece (27) has third and fourth ends,
the first and third ends are fixed at two sides of the insertion opening (22), respectively, at a distance greater than the width of the gene sequencing chip (1);
the second and fourth ends are movable with a reset distance smaller than the width of the gene sequencing chip (1),
the first and third ends are located at an upstream end along an insertion direction, and
the second and fourth ends are located at a downstream end of along the insertion direction.

10. The combination according to any one of claims 7 to 9, wherein the mounting frame (20) further comprises fabrication holes (29), correspondingly located right above the protrusions (26) and configured to allow the protrusions (26) to be injection molded.

## Patentansprüche

1. Gensequenzierungschip (1), umfassend einen Siliziumwafer (2) und eine durchsichtige Abdeckplatte (3), die an dem Siliziumwafer (2) anhaftet, wobei der Siliziumwafer (2) bereitgestellt ist mit
einem Adaptergebiet (18), das einen Adapter enthält, der im Stande ist, ein Genmolekül einzufangen, das zu sequenzieren ist, und
einen Flüssigkeitseinlass (7) und einen Flüssigkeitsauslass (8), die einen Flusskanal (6) nach außen verbinden;
wobei der Gensequenzierungschip (1) **dadurch gekennzeichnet ist, dass**:
die durchsichtige Abdeckplatte (3) mit einer Flusskanalkerbe (4) und einer Dichtungsmittelkerbe (5) an einer Oberfläche, wo die durchsichtige Abdeckplatte (3) an dem Siliziumwafer (2) anhaftet, gebildet ist,
wobei die Flusskanalkerbe (4) und der Siliziumwafer (2) den Flusskanal (6) umschließen, der einer Flüssigkeit erlaubt durchzufließen und das Adaptergebiet (18) beherbergt, und
wobei die Dichtungsmittelkerbe (5) und der Siliziumwafer (2) einen Dichtungsmittelkanal (9) umschließen, der mit einem Dichtungsmittel (10) gefüllt ist und den Flusskanal (6) umgibt,
wobei der Flusskanal (6), in einer langen Streifenform, einen Mittelbereich (11) und einen ersten und einen zweiten Abschlussbereich (12), die an beziehungsweise verbunden mit zwei Enden des Mittelbereichs (11) liegen; wobei der Flüssigkeitseinlass (7) und der Flüssigkeitsauslass (8) bei dem ersten beziehungsweise zweiten Abschlussbereich (12) liegen; und wobei
der Siliziumwafer (2) weiter mit einer Ausbuchtung (13) bereitgestellt ist, die bei dem Mittelbereich (11) des Flusskanals (6) liegt und auf der das Adaptergebiet (18) bereitgestellt ist.

2. Gensequenzierungschip (1) nach Anspruch 1, wobei der erste und der zweite Abschlussbereich (12) jeweils in einer Form sind, dass sie scharfwinklig nach außen vorragen.

3. Gensequenzierungschip (1) nach Anspruch 2, wobei
der nach außen vorragende scharfe Winkel in einem Grad von 60° eingeschlossen und mit einem Eckradius von 2 mm abgefast ist;
der erste und der zweite Abschlussbereich (12) jeweils mit dem Mittelbereich (11) durch einen abgerundeten Übergang mit einem Eckradius von 2 mm verbunden sind;
der Flusskanal (6) eine Länge von 69,8 mm, eine Breite von 5 mm für den Mittelbereich (11) und einen Abstand von einer oberen Oberfläche der Ausbuchtung (13) zu einer Bodenoberfläche der Flusskanalkerbe (4) von 0,05±0,005 mm aufweist; und
der Flüssigkeitseinlass (7) und der Flüssigkeitsauslass (8), die jeweils als ein rundes Loch mit einem Durchmesser von 0,8 mm gebildet sind, voneinander bei einem Mittenabstand von 69 mm auseinanderliegen.

4. Gensequenzierungschip (1) nach einem der Ansprüche 1 bis 3, wobei
die durchsichtige Abdeckungsplatte (3) weiter mit einer Verstärkungsmittelkerbe (14) an der Oberfläche gebildet ist, wo die durchsichtige Abdeckungsplatte (3) an dem Siliziumwafer (2) anhaftet,
die Verstärkungsmittelkerbe (14) und der Siliziumwafer (2) einen Verstärkungsmittelkanal (15) umschließen, der mit einem Verstärkungsmittel (16) gefüllt ist und den Dichtungsmittelkanal (9) umgibt, und
der Siliziumwafer (2) weiter mit einem Luftloch (17) bereitgestellt ist, das zwischen dem Verstärkungsmittelkanal (15) und dem Dichtungsmittelkanal (9) liegt.

5. Gensequenzierungschip (1) nach Anspruch 4, wobei das Dichtungsmittel (10) und das Verstärkungsmittel (16) aus Polyurethankleber, Heißschmelzkleber und ultravioletthärtendem Kleber ausgewählt sind.

6. Gensequenzierungschip (1) nach einem der Ansprüche 1 bis 5, wobei
das Adaptergebiet (18) in einer runden Form mit einem Durchmesser von 220 nm ist, und
der Siliziumwafer (2) mit einer Vielzahl von den Adaptergebieten (18) in einem Array mit einem Mittenabstand von 900 nm zwischen angrenzenden zwei Adaptergebieten (18) bereitgestellt ist.

7. Kombination eines Montagerahmens (20) für den Gensequenzierungschip (1) und des Gensequenzierungschips (1) nach einem der Ansprüche 1 bis 6, **gekennzeichnet durch**
eine Montagekerbe (21), die dazu ausgebildet ist, den Gensequenzierungschip (1) zu beherbergen und zu diesem zu passen, und die mit einer Einsatzöffnung (22) bereitgestellt ist, um den Gensequenzierungschip (1) an einem Ende der Montagekerbe (21) aufzunehmen;
eine Löseschutzstruktur (23), die an der Einsatzöffnung (22) eingerichtet ist und dazu ausgebildet ist, den Gensequenzierungschip (1) daran zu hindern, aus der Einsatzöffnung (22) zu rutschen;
ein offenes Gebiet (24), das direkt über der Montagekerbe (21) liegt und dazu ausgebildet ist, Beobachtung des Flusskanals (6) des Gensequenzierungschips (1), der in der Montagekerbe (21) beherbergt ist, von außen zu erlauben; und
ein Positionierungsloch (25), das dazu ausgebildet ist, dem Montagerahmen (20) zu erlauben, auf einer äußeren Plattform positioniert zu werden.

8. Kombination nach Anspruch 7, wobei die Montagekerbe (21) mit symmetrischen Fortsätzen (26) an Innenseitenwänden bereitgestellt ist, die dazu ausgebildet sind, den Gensequenzierungschip (1) innerhalb der Montagekerbe (21) einzugrenzen.

9. Kombination nach Anspruch 7 oder 8, wobei
die Löseschutzstruktur (23) erste und zweite elastische Teile (27) umfasst, die symmetrisch an der Einsatzöffnung (22) eingerichtet sind,
das erste elastische Teil (27) ein erstes und ein zweites Ende aufweist,
das zweite elastische Teil (27) ein drittes und ein viertes Ende aufweist,
das erste und das dritte Ende an zwei Seiten der Einsatzöffnung (22) befestigt sind, beziehungsweise in einem größeren Abstand als der Breite des Gensequenzierungschips (1);
das zweite und das vierte Ende mit einem kleineren Rückstellabstand als der Breite des Gensequenzierungschips (1) beweglich sind,
das erste und das dritte Ende an einem stromaufwärtigen Ende entlang einer Einsatzrichtung liegen, und
das zweite und das vierte Ende an einem stromabwärtigen Ende entlang der Einsatzrichtung liegen.

10. Kombination nach einem der Ansprüche 7 bis 9, wobei der Montagerahmen (20) weiter Fertigungslöcher (29) umfasst, die entsprechend direkt über den Fortsätzen (26) liegen und dazu ausgebildet sind, den Fortsätzen (26) zu erlauben, spritzgegossen zu werden.

## Revendications

1. Puce de séquençage de gène (1), comprenant une tranche de silicium (2) et une feuille de protection à transmission de lumière (3) qui adhère à la tranche de silicium (2), la tranche de silicium (2) est pourvue d'
une région d'adaptateur (18) contenant un adaptateur qui est capable de capturer une molécule de gêne à séquencer, et
une entrée de liquide (7) et une sortie de liquide (8) reliant un canal d'écoulement (6) avec l'extérieur ; ladite puce de séquençage de gène (1) est **caractérisée en ce que** :
la feuille de protection à transmission de lumière (3) est formée avec une rainure de canal d'écoulement (4) et une rainure d'étanchéité (5) au niveau d'une surface où la feuille de protection à transmission de lumière (3) adhère à la tranche de silicium (2),
dans laquelle la rainure de canal d'écoulement (4) et la tranche de silicium (2) enferment le canal d'écoulement (6) qui permet à un liquide de s'écouler à travers et loge la région d'adaptateur (18), et
dans laquelle la rainure d'étanchéité (5) et la tranche de silicium (2) enferment un canal d'étanchéité (9) qui est rempli d'un agent d'étanchéité (10) et entoure le canal d'écoulement (6),
dans laquelle le canal d'écoulement (6), dans une forme de bande longue, comprend une section de milieu (11) et des première et seconde sections terminales (12) situées au niveau de et reliées à deux extrémités de la section de milieu (11), respectivement ; dans laquelle l'entrée de liquide (7) et la sortie de liquide (8) sont situées au niveau des première et seconde sections terminales (12), respectivement; et dans laquelle
la tranche de silicium (2) est pourvue en outre d'un bossage (13) situé au niveau de la section de milieu (11) du canal d'écoulement (6) et sur lequel la région d'adaptateur (18) est prévue.

2. Puce de séquençage de gène (1) selon la revendication 1, dans laquelle les première et seconde sections terminales (12) sont chacune dans une forme d'un angle aigu faisant saillie vers l'extérieur.

3. Puce de séquençage de gène (1) selon la revendication 2, dans laquelle
l'angle aigu faisant saillie vers l'extérieur est dans un degré inclus de 60° et chanfreiné avec un rayon de pointe de 2 mm ;
les première et seconde sections terminales (12) sont chacune connectées à la section de milieu (11) via une transition arrondie avec un rayon de pointe de 2 mm ;
le canal d'écoulement (6) est d'une longueur de 69,8 mm, d'une largeur de 5 mm pour la section de milieu (11), et d'une distance à partir d'une surface supérieure du bossage (13) jusqu'à une surface inférieure de la rainure de canal d'écoulement (4) de 0,05±0,005 mm ; et
l'entrée de liquide (7) et la sortie de liquide (8), chacune formées en tant que trou arrondi dans un diamètre de 0,8 mm, sont à l'écart l'une de l'autre à une distance centrale de 69 mm.

4. Puce de séquençage de gène (1) selon l'une quelconque des revendications 1 à 3, dans laquelle
la feuille de protection à transmission de lumière (3) est en outre formée avec une rainure d'agent de renforcement (14) au niveau de la surface où la feuille de protection à transmission de lumière (3) adhère à la tranche de silicium (2),
la rainure d'agent de renforcement (14) et la tranche de silicium (2) enferment un canal d'agent de renforcement (15) qui est rempli d'un agent de renforcement (16) et entoure le canal d'étanchéité (9), et
la tranche de silicium (2) est en outre pourvue d'un trou d'air (17) qui est situé entre le canal d'agent de renforcement (15) et le canal d'étanchéité (9).

5. Puce de séquençage de gène (1) selon la revendication 4, dans laquelle l'agent d'étanchéité (10) et l'agent de renforcement (16) sont sélectionnés parmi un adhésif en polyuréthane, un adhésif thermoplastique et un adhésif à durcissement par ultraviolet.

6. Puce de séquençage de gène (1) selon l'une quelconque des revendications 1 à 5, dans laquelle
la région d'adaptateur (18) est dans une forme arrondie avec un diamètre de 220 nm, et
la tranche de silicium (2) est pourvue d'une pluralité des régions d'adaptateur (18) agencées dans un rayon avec une distance centrale de 900 nm entre deux régions d'adaptateur adjacentes (18).

7. Combinaison d'un cadre de montage (20) pour la puce de séquençage de gène (1) et la puce de séquençage de gène (1) selon l'une quelconque des revendications 1 à 6, étant **caractérisée par**
une rainure de montage (21), configurée pour loger et correspondre à la puce de séquençage de gène (1) et pourvue d'une ouverture d'insertion (22) pour recevoir la puce de séquençage de gène (1) au niveau d'une extrémité de rainure de montage (21) ;
une structure anti-retrait (23), agencée au niveau de l'ouverture d'insertion (22) et configurée pour empêcher la puce de séquençage de gène (1) de glisser hors de l'ouverture d'insertion (22) ;
une région ouverte (24), située juste au-dessus de la rainure de montage (21) et configurée pour permettre l'observation du canal d'écoulement (6) de la puce de séquençage de gène (1) logée dans la rainure de montage (21) depuis l'extérieur ; et
un trou de positionnement (25), configuré pour permettre au cadre de montage (20) d'être positionné sur une plateforme extérieure.

8. Combinaison selon la revendication 7, dans laquelle la rainure de montage (21) est pourvue de saillies symétriques (26) au niveau de parois latérales internes qui sont configurées pour restreindre la puce de séquençage de gène (1) au sein de la rainure de montage (21).

9. Combinaison selon la revendication 7 ou 8, dans laquelle
la structure anti-retrait (23) comprend des première et seconde pièces élastiques (27) qui sont agencées symétriquement au niveau de l'ouverture d'insertion (22),
la première pièce élastique (27) présente des première et deuxième extrémités,
la seconde pièce élastique (27) présente des troisième et quatrième extrémités,
les première et troisième extrémités sont fixées au niveau de deux côtés de l'ouverture d'insertion (22), respectivement, à une distance supérieure à la largeur de la puce de séquençage de gène (1) ;
les deuxième et quatrième extrémités sont mobiles avec une distance de réinitialisation plus petite que la largeur de la puce de séquençage de gène (1),
les première et troisième extrémités sont situées au niveau d'une extrémité en amont le long d'une direction d'insertion, et
les deuxième et quatrième extrémités sont situées au niveau d'une extrémité en aval le long de la direction d'insertion.

10. Combinaison selon l'une quelconque des revendications 7 à 9, dans laquelle le cadre de montage (20) comprend en outre des trous de fabrication (29), situés de façon correspondante juste au-dessus des saillies (26) et configurés pour permettre aux saillies (26) d'être moulées par injection.
